(19) **Europäisches Patentamt**
**European Patent Office**
**Office européen des brevets**

(11) **EP 2 842 521 B1**

(12) **EUROPEAN PATENT SPECIFICATION**

(45) Date of publication and mention
of the grant of the patent:
**22.02.2017 Bulletin 2017/08**

(51) Int Cl.:
*A61F 2/50* (2006.01)  *H02J 5/00* (2016.01)
*H02J 7/02* (2016.01)

(21) Application number: **14182320.3**

(22) Date of filing: **26.08.2014**

(54) **Wireless charging for prosthetic device**

Drahtloses Laden für prothetische Vorrichtung

Dispositif de charge sans fil pour prothèses

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.08.2013 US 201361870704 P
22.11.2013 US 201361907975 P
22.08.2014 US 201414466878**

(43) Date of publication of application:
**04.03.2015 Bulletin 2015/10**

(73) Proprietor: **Freedom Innovations, LLC
Irvine, California 92618 (US)**

(72) Inventor: **Haque, Kamran
Riverside, California 92505 (US)**

(74) Representative: **Cooley (UK) LLP
Dashwood
69 Old Broad Street
London EC2M 1QS (GB)**

(56) References cited:
**EP-A2- 1 868 275**    **US-A1- 2010 033 021**
**US-A1- 2011 060 421**    **US-A1- 2012 022 667**
**US-A1- 2012 161 544**

- **MOHAMAD SAWAN ET AL: "Multicoils-based
inductive links dedicated to power up implantable
medical devices: modeling, design and
experimental results", BIOMEDICAL
MICRODEVICES, vol. 11, no. 5, 2 June 2009
(2009-06-02), pages 1059-1070, XP055156390,
ISSN: 1387-2176, DOI:
10.1007/s10544-009-9323-7**
- **DUKJU AHN ET AL: "Effect of Coupling Between
Multiple Transmitters or Multiple Receivers on
Wireless Power Transfer", IEEE TRANSACTIONS
ON INDUSTRIAL ELECTRONICS, IEEE SERVICE
CENTER, PISCATAWAY, NJ, USA, vol. 60, no. 7,
1 July 2013 (2013-07-01), pages 2602-2613,
XP011495393, ISSN: 0278-0046, DOI:
10.1109/TIE.2012.2196902**

## Description

### FIELD

The present disclosure relates to prosthetic devices. More particularly, the present disclosure relates to charging prosthetic devices.

### BACKGROUND

Many modem prosthetic devices are electrically powered to provide actuation or damping of the prosthetic device. While such powered prosthetic devices can provide a more natural motion, the mobile nature of prosthetic devices generally requires the use of a power storage unit such as a rechargeable battery to power the prosthetic device. Charging the power storage unit usually involves plugging a power supply into the prosthetic device. While the power storage unit charges, movement of the prosthetic device is restrained by a cable connected to the power supply or the prosthetic device must be removed. Plugging a power supply into the prosthetic device also typically requires a power input jack on the prosthetic device which can compromise the prosthetic device's resistance to environmental conditions such as dirt, moisture and water. In addition, charging a prosthetic device using a power input jack may require removal of an outer skin or a hole in an outer skin in order to access the power input jack. The outer skin can enclose the prosthetic device to provide a more natural and aesthetic appearance. Removing the outer skin or providing a hole in the outer skin adversely affects the aesthetic appearance of the device or can require additional effort in removing the outer skin.

US 20110060421 details a vacuum pump system for a prosthesis device including a suspension mechanism and co-operates with a pressure source, an adaptor assembly, circuitry, and a power source. US20120161544 details a wireless power transmission device that supplies power to a receiving device including a second that supplies power to a power receiving device including a second self-resonant coil having a winding structure in which a conductive wire is wound one turn or more perpendicular to a second central axis.

### SUMMARY

The invention is defined by the independent claim and preferred features are set out in the dependent claims. In view of the foregoing, the present disclosure involves wirelessly charging a prosthetic device via magnetic coupling. To further improve the freedom of movement of the prosthetic device while charging, some aspects of the present disclosure involve wirelessly charging a prosthetic device using resonant magnetic coupling. Traditional magnetic induction methods of charging devices typically rely on a tight coupling between transmitter and receiver coils to maintain a power transfer efficiency. Resonant magnetic coupling can allow for a farther distance between transmitter and receiver coils so as to improve the freedom of movement while charging and to allow for the simultaneous charging of multiple prosthetic devices.

According to one embodiment, a prosthetic device includes a power storage unit to power the prosthetic device and an electromagnetic receiver including a plurality of coils arranged about a portion of the prosthetic device. The electromagnetic receiver is configured to receive a magnetic field from an electromagnetic transmitter magnetically coupled with the electromagnetic receiver and to generate electric power from the magnetic field. Circuitry of the prosthetic device is configured to store the electric power generated from the magnetic field in the power storage unit.

By arranging a plurality of coils about a portion of the prosthetic device, it is ordinarily possible to allow for charging from different angles between the prosthetic device and the electromagnetic transmitter. In some embodiments, the magnetic field is a resonating magnetic field with a resonant frequency of the electromagnetic receiver.

According to another embodiment, the present disclosure includes an electromagnetic transmitter including circuitry configured to receive electric power from a power supply. A plurality of coils of the electromagnetic transmitter is configured to generate a magnet field using the electric power to magnetically couple with an electromagnetic receiver of a prosthetic device. In one aspect, the electromagnetic transmitter is further configured to generate a resonating magnetic field with a resonant frequency of the electromagnetic receiver of the prosthetic device.

### BRIEF DESCRIPTION OF THE DRAWINGS

The features and advantages of the embodiments of the present disclosure will become more apparent from the detailed description set forth below when taken in conjunction with the drawings. The drawings and the associated descriptions are provided to illustrate embodiments of the disclosure and not to limit the scope of what is claimed.

FIG. 1 is a block diagram depicting wireless charging of a prosthetic device according to an embodiment.
FIG. 2 illustrates a prosthetic device including an electromagnetic receiver according to an embodiment.

FIG. 3 is a front view of an electromagnetic receiver including adjacent coils according to an embodiment.

FIG. 4 is a side view of an electromagnetic receiver with overlapping flexible circuits according to an embodiment.

FIG. 5 illustrates a prosthetic device charging system with multiple electromagnetic transmitters according to an embodiment.

FIG. 6 illustrates a portable electromagnetic transmitter inside a car according to an embodiment.

FIG. 7 is a front view of an electromagnetic transmitter with partially overlapping coils according to an embodiment.

FIG. 8 is a side view of an electromagnetic transmitter with overlapping flexible circuits according to an embodiment.

FIG. 9 is a flowchart for a charging process performed by an electromagnetic transmitter according to an embodiment.

FIG. 10 is a flowchart for a charging process performed by a prosthetic device according to an embodiment.

## DETAILED DESCRIPTION

In the following detailed description, numerous specific details are set forth to provide a full understanding of the present disclosure. It will be apparent, however, to one of ordinary skill in the art that the various embodiments disclosed may be practiced without some of these specific details. In other instances, well-known structures and techniques have not been shown in detail to avoid unnecessarily obscuring the various embodiments.

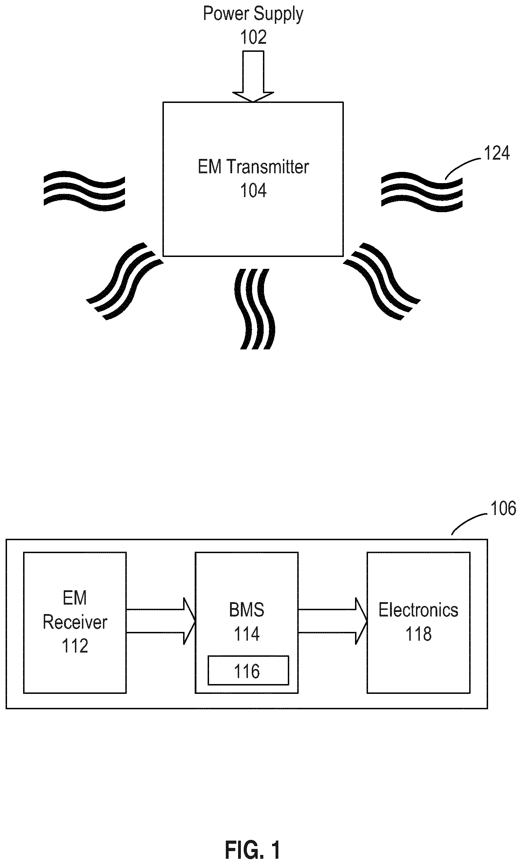

FIG. 1 depicts wireless charging of prosthetic device 106 using electromagnetic (EM) transmitter 104. Prosthetic device 106 can be, for example, a battery powered prosthetic joint such as a prosthetic ankle or knee, or a prosthetic leg including both a prosthetic ankle and knee.

EM transmitter 104 is powered by power supply 102 and is configured to transmit magnetic field 124 to EM receiver 112 of prosthetic device 106. As will be discussed in more detail below, power supply 102 can be an alternating current (AC) power supply (e.g., from a wall outlet) or a direct current (DC) power supply (e.g., from a battery or wall power adapter).

In the example of FIG. 1, EM transmitter is further configured to transmit magnetic field 124 as a resonating magnetic field at a resonant frequency of EM receiver 112 of prosthetic device 106. In some embodiments, such a resonant frequency can be within a range of 100 kHz and 10 MHz.

In one implementation, each of EM transmitter 104 and EM receiver 112 can include a plurality of coils or inductors electrically connected to one or more tuning capacitors for tuning to a frequency, f, which can be represented as shown in Equation 1 below:

$$ f = \frac{1}{2\pi\sqrt{LC}} \qquad\qquad \text{Equation 1} $$

where L is an inductance of the plurality of coils at resonance and C is a capacitance of the at least one tuning capacitor for the plurality of coils. Power transfer efficiency through resonance can be improved by reducing resistance in the transmitting or receiving coils.

In some implementations, EM transmitter 104 can include different inductors and/or capacitors for generating magnetic fields at different frequencies. In this regard, the tuning capacitor can include a variable capacitor for tuning to different frequencies. In yet other implementations, EM transmitter 104 can include a chipset or integrated circuit for generating a magnetic field.

EM transmitter 104 can also include circuitry for communicating with prosthetic device 106 or controlling operation of EM transmitter 104. Such circuitry can include, for example, a controller, a processor, wireless communication chipset, or an application-specific integrated circuit (ASIC) which executes computer-readable instructions stored in a memory of EM transmitter 104.

As shown in FIG. 1, prosthetic device 106 includes EM receiver 112, battery management system (BMS) 114, and electronics 118, each of which is carried by prosthetic device 106. EM receiver 112 is configured to receive magnetic field 124 from EM transmitter 104 and to generate electric power from magnetic field 124. The power generated over time is proportional to the strength of the magnetic field. EM receiver 112 includes a plurality of inductors or coils which convert magnetic field 124 into an electric field to generate electric power. The plurality of coils can be electrically connected to one or more tuning capacitors to tune to a frequency used by EM transmitter 104. In yet other implementations, EM receiver 112 includes a chipset or integrated circuit for receiving magnetic field 124 and converting magnetic field 124 into an electric field to generate electric power.

EM receiver 112 can also include circuitry for controlling operation of EM receiver 112. Such circuitry can include, for example, a controller, a processor, a wireless communication chipset, or an ASIC for executing computer-readable instructions stored in a memory of prosthetic device 106.

Although inductive chargers, such as those used for electric toothbrushes, can provide wireless charging, such inductive charging systems generally require that the power transmitter and the power receiver are spatially aligned with each other. This would require a user of a prosthetic device to remove the prosthetic device for charging or keep the prosthetic device in a fixed position while charging. As with wired charging, keeping the prosthetic device in a fixed position would be cumbersome for the user of the prosthetic device as it limits mobility of the prosthetic device and introduces charge time inefficiencies when the transmitter and the receiver are not properly aligned.

By tuning EM transmitter 104 and EM receiver 112 to approximately the same resonant frequency, EM transmitter 104 and EM receiver 112 do not need to be closely aligned and the distance between them can be increased so that EM transmitter 104 can be remote from prosthetic device 106 while still transferring power to prosthetic device 106. In some implementations, the amount of distance between EM transmitter 104 and EM receiver 112 can vary from several inches to over ten feet. Moreover, it is ordinarily possible to transfer power to prosthetic device 106 without having to remove prosthetic device 106 or restrict a user's movement of prosthetic device 106. In addition, EM resonant wireless charging can allow for simultaneous charging of multiple prosthetic devices, which can be especially useful for users with multiple prosthetic devices.

In some implementations, circuitry of EM transmitter 104 can adjust an amount of electric power used from power supply 102 to dynamically adjust for changes in the position of prosthetic device 106 or to dynamically adjust to charging additional devices while maintaining a real-time communication link. In another implementation, EM transmitter 104 may use between 10 and 20 Watts from power supply 102 to generate magnetic field 124. EM transmitter 104 may then vary the amount of power between 10 and 20 Watts based on a reflected power in magnetic field 124 that is not received by EM receiver 112 and is reflected back to EM transmitter 104.

A decrease in the reflected power can indicate that more devices are being charged or that the positioning of prosthetic device 106 has changed such that more of the transmitted power is received by EM receiver 112. In such an example, EM transmitter 104 may then increase the power used from power supply 102 toward an upper power limit so as to transfer more power via magnetic field 124.

On the other hand, an increase in the reflected power reflected back to EM transmitter 104 can indicate that less of the transmitted power is being received. In one implementation, if the reflected power exceeds a threshold, EM transmitter 104 may first increase the power used from power supply 102 to increase a range of magnetic field 124. If the proportion of reflected power to transmitted power does not decrease after increasing the power used, EM transmitter 104 may then determine that prosthetic device 106 is no longer within a range to efficiently receive magnetic field 124. EM transmitter 104 may then stop generating magnetic field 124 and enter a low power or standby state.

Adjustments to the power used to generate magnetic field 124 can also be made based on digital communications between EM transmitter 104 and prosthetic device 106 using a wireless communications link such as, for example, a Bluetooth Low Energy or a wireless Ethernet communications link. In this regard, each of EM transmitter 104 and prosthetic device 106 can include a wireless communication module or chipset so that EM transmitter 104 can adjust a frequency or a power used to generate magnetic field 124 based on information received from prosthetic device 106 concerning a location or charging efficiency of EM receiver 112.

In some implementations, EM transmitter 104 and EM receiver 112 may also operate in accordance with a particular wireless charging standard, such as Qualcomm's WiPower standard, A4WP's Rezence standard, or the Wireless Power Consortium's Qi standard.

As shown in the example of FIG. 1, prosthetic device 106 includes BMS 114 which includes power storage unit 116 that can, for example, include a rechargeable battery or super capacitor capable of storing power. BMS 114 may also include circuitry for storing power generated from magnetic field 124 in power storage unit 116. Such circuitry can include a full wave rectifier and a regulator circuit to convert AC power generated from magnetic field 124 into DC power for charging power storage unit 116.

Electronics 118 can include controls for actuation and/or damping of prosthetic device 106 and electronics for communication with other devices. In this regard, electronics 118 can include at least one of a motor, a valve, a sensor, or a controller for actuating or damping a movement of prosthetic device 106.

In one implementation, electronics 118 also includes an antenna for receiving a radio frequency (RF) beacon transmitted from EM transmitter 104. In such an implementation, EM transmitter 104 can periodically transmit beacons and electronics 118 can respond by transmitting device information to EM transmitter 104. The communication between EM transmitter 104 and electronics 118 may be in accordance with a particular communications protocol such as Bluetooth. The device information can indicate different frequencies at which EM receiver 112 can tune to for receiving power from EM transmitter 104 via magnetic field 124. EM transmitter 104 may then select a frequency to tune to based on the device information received from prosthetic device 106.

In other implementations, the device information may include information about prosthetic device 106 such as a proximity or alignment indication for EM receiver 112 with respect to EM transmitter 104, an average power usage rate, or information about BMS 114, such as at least one of a charging efficiency, a state of charge, a charge capacity, and an average or estimated charge time. EM transmitter 104 may use this device information to adjust the rate at which power is transferred to EM receiver 112 by changing the amount of power used from power supply 102 to generate magnetic field 124. For example, if the device information indicates that the current charge level is fully charged, EM transmitter 104 may select a lower rate or power at which to transfer power to EM receiver 112. In another example, if the device information indicates a long estimated charge time, EM transmitter 104 may select a higher rate or power at which to transfer power to EM receiver 112.

In some implementations, the device information may be wirelessly transmitted to a mobile device such as a cellular phone or tablet to allow an application on the mobile device to display prosthetic device information to a user. Such prosthetic device information can include information concerning a proximity or alignment of EM receiver 112 with respect to EM transmitter 104, an average power usage rate, a charging efficiency, a state of charge, a charge capacity, and an average or estimated charge time.

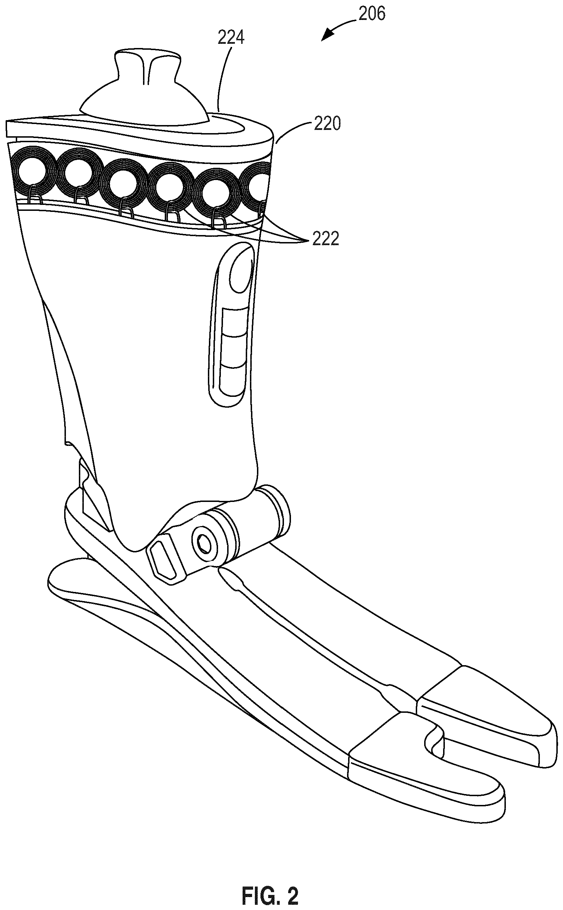

FIG. 2 illustrates an example of a prosthetic device including an electromagnetic receiver according to an embodiment. In the example of FIG. 2, prosthetic device 206 includes a prosthetic ankle joint and a prosthetic foot. As discussed above, wireless charging of prosthetic device 206 can reduce the need for a power input which can allow dirt and moisture into prosthetic device 206. In addition, a substantially uniform outer layer can be placed around prosthetic device 206 for a more natural appearance without requiring any holes for a power input or requiring removal of the outer layer for charging.

In the example embodiment of FIG. 2, EM receiver 220 is located about a top portion 224 of prosthetic device 206. In other embodiments, EM receiver 220 may be placed about different portions of prosthetic device 206 such as along a sole portion of the foot or around a portion of prosthetic device 206 closer to the ankle joint.

EM receiver 220 includes a plurality or array of coils 222 that are arranged adjacent to one another so that the diameters of coils 222 completely surround portion 224 of prosthetic device 206. As shown in FIG. 2, each coil 222 of the plurality of coils is in physical contact with another coil 222 and forms a ring that completely surrounds portion 224.

By arranging coils 222 about portion 224, it is ordinarily possible to increase the freedom of motion of prosthetic device 206 while charging since EM receiver 220 is capable of receiving a magnetic field from different angles. In other words, the rotation or angle of prosthetic device 206 may change with respect to an EM transmitter while charging since different coils 222 may be used in varying degrees depending upon the relative position of the coil with respect to the EM transmitter. The use of multiple coils 222 can also increase the amount of electric power generated from the magnetic field by providing for better magnetic coupling with the EM transmitter.

As shown in FIG. 2, coils 222 partially overlap each other to further improve a power transfer efficiency of EM receiver 220 since coils 222 cover all angles around portion 224. In other embodiments, coils 222 may only touch on their edges as opposed to overlapping or EM receiver 220 may include small gaps between coils 222. In yet other embodiments, prosthetic device 106 may include bands of coils 222 at different heights along prosthetic device 206 so as to allow for placement of an EM transmitter at different heights while charging.

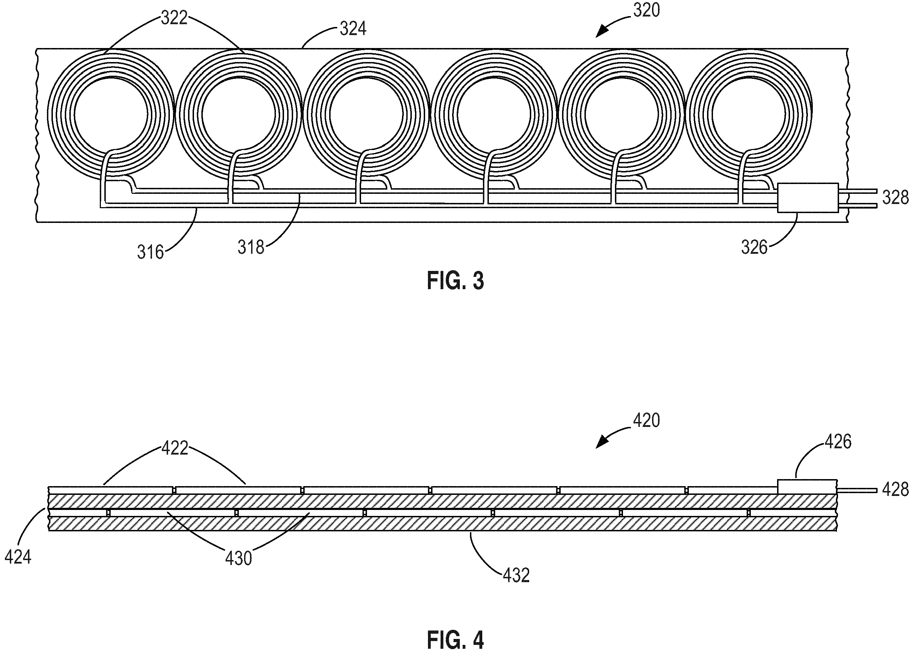

FIG. 3 provides a front view of EM receiver 320 where coils 322 are arranged substantially in the same plane with each coil 322 adjacent to another coil 322 so that coils 322 touch one another. Each coil 322 can include a printed circuit board (PCB) trace along flexible circuit 324 or a flexible wire mounted on flexible circuit 324. This can generally allow EM receiver 320 to be flexible enough to wrap around a portion of the prosthetic device.

EM receiver 320 also includes circuitry 326 which is configured to store electric power generated by coils 322 in a power storage unit. Circuitry 326 is electrically connected to each of coils 322 via traces 316 and 318. Electric power generated by coils 322 travels along traces 316 and 318 to circuitry 326, which can include a summing circuitry to add the electric power generated by coils 322 before storing the electric power in a power storage unit via power output 328. In some embodiments, circuitry 326 can also include a full wave rectifier or a regulator circuit to convert AC power into DC power for charging a power storage unit.

FIG. 4 provides a side view of EM receiver 420 including overlapping flexible circuits 424 and 432 according to an embodiment. As shown in FIG. 4, EM receiver 420 includes a top plurality of coils 422 and a bottom plurality of coils 430 each arranged on flexible circuits 424 and 432, respectively. Other embodiments may include more than the two layers of flexible circuits shown in FIG. 4.

Although there is a small lateral gap between each coil of coils 422 and each coil of coils 430, the coils are arranged such that the coils of flexible circuit 422 are laterally offset from the coils of flexible circuit 432 so as to provide increased coverage for receiving a magnetic field. The coils of both flexible circuits may be connected to one another using the same traces on one of the flexible circuits may or may use separate traces or wiring.

EM receiver 420 of FIG. 4 also includes circuitry 426 which may include a summing circuitry for adding the electric power generated by coils 422 and 430 before storing the electric power in a battery storage unit via power output 428. In some embodiments, circuitry 426 can also include a full wave rectifier or a regulator circuit to convert AC power into DC power for charging a power storage unit.

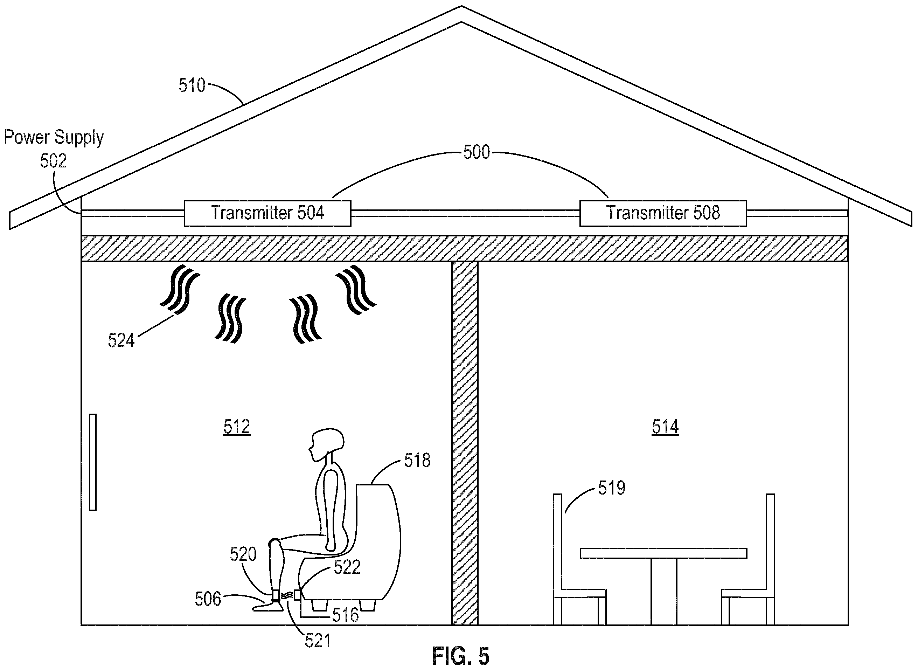

FIG. 5 illustrates prosthetic device charging system 500 including EM transmitters 504 and 508 according to an embodiment. EM transmitters 504 and 508 can have a construction similar to EM transmitter 104 of FIG. 1 and are powered by power supply 502, which can be a power distribution system for building 510.

Each of EM transmitters 504 and 508 is constructed to secure to a building structure and placed in relation to a different area of building 510. In particular, EM transmitter 504 is placed above room 512 of building 510 and transmitter 508 is placed above room 514 of building 510. By locating EM transmitters 504 and 508 in different areas of building 510, a user of prosthetic device 506 can continue to charge prosthetic device 506 even when they move from room 512 to room 514, or vice-versa. In this regard, EM transmitters can be strategically placed within a building to allow for continuous charging of a prosthetic device or devices as a user moves throughout the building.

Although the embodiment of FIG. 5 shows EM transmitters 504 and 508 above a ceiling, other embodiments can include EM transmitters 504 and 508 inside rooms 512 and 514, such as mounted on an interior wall surface of rooms 512 and 514 or beneath furniture in rooms 512 and 514 such as a chair. The placement of EM transmitters 504 and 508 can be made to improve a power transfer efficiency based on a likely location of an EM receiver in a particular room and EM transmitters 504 and 508 may or may not be visible from within the room. In addition, the locations of EM transmitters 504 and 508 do not need to be over the room as shown in FIG. 5. In other embodiments, EM transmitters 504 and 508 can be strategically placed in other locations for power transfer efficiency such as below a floor or within a wall.

In addition to prosthetic device charging system 500 including EM transmitters 504 and 508, FIG. 5 also includes portable EM transmitter 522 mounted or secured on chair 518. Portable EM transmitter 522 may charge prosthetic device 506 in addition to EM transmitter 504 or EM transmitter 508 to provide for quicker charging. Portable EM transmitter 522 can be detachably secured to chair 518 using, for example, Velcro, a magnet, a strap, or a clip, so as to allow portable EM transmitter 522 to be repositioned or located elsewhere, such as on chair 519 in room 514. In the example of FIG. 5, portable EM transmitter 522 includes power supply 516 which may be connected to an outlet in room 512.

In the example of FIG. 5, prosthetic device 506 is charged by EM transmitter 504 via resonating magnetic field 524 while also being charged by portable EM transmitter 522 via resonating magnetic field 521. EM receiver 520 of prosthetic device 506 is magnetically coupled with EM transmitter 504 and portable EM transmitter 522 at a resonant frequency of EM receiver 520 so that EM receiver 520 is not required to be closely aligned with EM transmitter 504 or portable EM transmitter 522 to receive power via magnetic fields 524 and 521. Accordingly, a user of prosthetic device 506 is able to move prosthetic device 506 while it charges.

In the example of FIG. 5, EM transmitter 508 is not transmitting a magnetic field. In this regard, charging system 500 may determine by comparing reflected powers received at EM transmitters 504 and 508 that prosthetic device 506 is closer to EM transmitter 504 than to EM transmitter 508. In other implementations, charging system 500 may use a digital wireless communications link to determine a relative location of EM receiver 520. Charging system 500 may then place EM transmitter 508 into a low power or standby state where no magnetic field is generated by EM transmitter 508. In other embodiments, EM transmitters 504 and 508 may each continuously generate magnetic fields regardless of whether the magnetic fields are received by EM receiver 520.

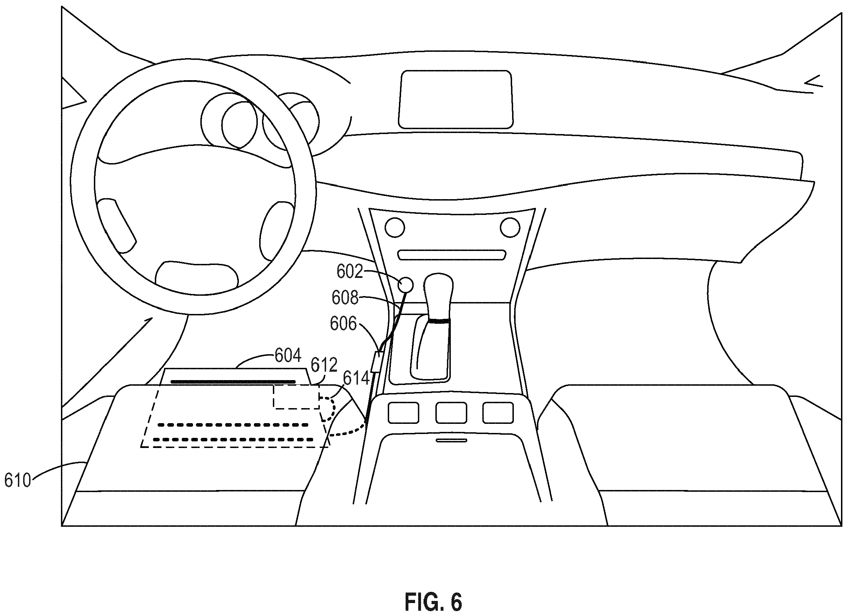

FIG. 6 illustrates portable EM transmitters 604 and 612 according to an embodiment. As shown in FIG. 6, portable EM transmitter 604 is in the form of a mat that can be plugged into power supply 602, which in the example of FIG. 6, is a cigarette lighter in the interior of an automobile. EM transmitter 604 is connected to power supply 602 via power cable 608, which is securely routed with clip 606 to avoid interference with operation of the automobile. In other embodiments, portable EM transmitter 604 can include a wall plug for obtaining power from a power outlet.

Portable EM transmitter 612 is secured onto a portion of the car seat and is electrically connected to portable EM transmitter 604 to receive power from power supply 602 via portable EM transmitter 604. This arrangement of transmitters can provide for charging coverage in both a horizontal direction with EM transmitter 604 and in a vertical direction with EM transmitter 612.

EM transmitter 612 may be detachably secured onto the interior of the automobile using, for example, Velcro, a magnet, a strap, or a clip. Both EM transmitters 604 and 612 can be moved to different locations such as to different areas of the automobile, a different automobile, or to different locations at an office or home. Other embodiments may include only one of portable EM transmitter 604 or 612 without the other.

As with EM transmitters 104, 504, 508, and 522, portable EM transmitters 604 and 612 include a plurality or array of coils for generating a magnetic field to magnetically couple with an EM receiver of a prosthetic device. By tuning EM transmitters 604 and 612 to a resonant frequency of the EM receiver, the prosthetic device can wirelessly charge while allowing movement of the prosthetic device.

In the example of FIG. 6, EM transmitter 604 is located mostly below seat 610 to allow the driver to wirelessly charge a prosthetic device while driving. In other implementations, EM transmitter 604 can be placed in other locations such as on a back of seat 610 to allow for charging by users in different seats such as the back seat.

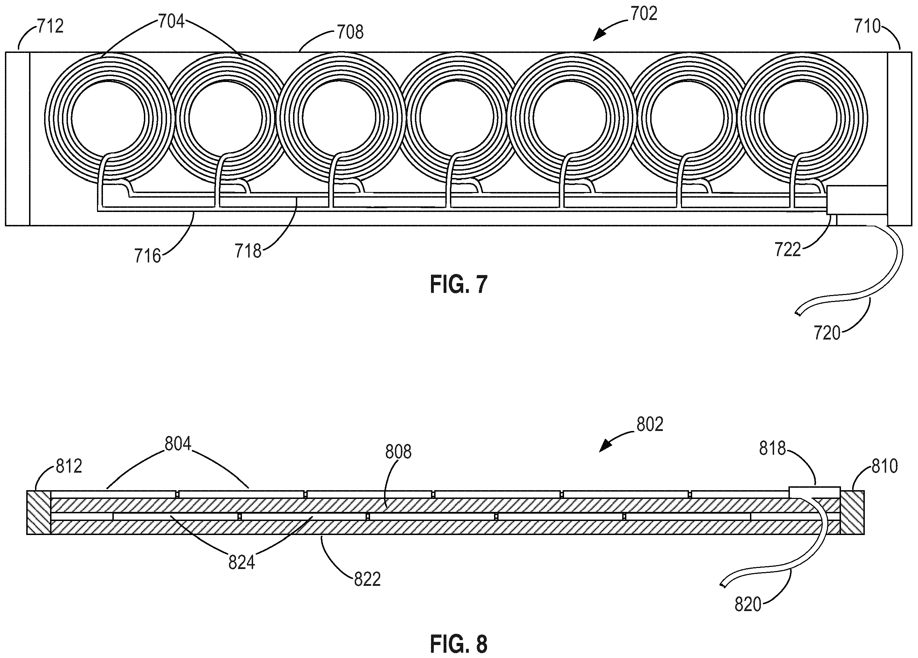

FIG. 7 provides a front view of EM transmitter 702 capable of being secured onto a prosthetic device. EM transmitter 702 includes flexible circuit 708 which can allow for EM transmitter 702 to be wrapped around the prosthetic device. By locating EM transmitter 702 next to an EM transmitter of a prosthetic device, it is ordinarily possible to provide quicker charging of the prosthetic device due to the decreased distance between EM transmitter 702 and the EM receiver.

As shown in FIG. 7, EM transmitter 702 is configured as a belt that can be wrapped around an exterior portion of a prosthetic device such as prosthetic device 206 in FIG. 2. In more detail, attachment portions 710 and 712 allow EM transmitter 702 to form a loop that can be worn around the prosthetic device. Attachment portions 710 and 712 can include Velcro, a magnet, a clip, a strap, a buckle, or other ways of securing EM transmitter 702 onto itself.

In one embodiment, one or both of attachment portions 710 and 712 can include a magnet that can be used to secure EM transmitter 702 onto a prosthetic device. The magnet may also be used to properly align EM transmitter 702 laterally or vertically onto the prosthetic device by securing EM transmitter 702 onto a corresponding magnet located near an EM receiver of the prosthetic device. Such alignment of EM transmitter 702 can help to ensure a more efficient alignment of coils 704 with respect to the coils of an EM receiver of the prosthetic device. In other embodiments, EM transmitter 702 can use other alignment indicators to indicate when EM transmitter 702 is properly aligned with respect to an EM receiver of the prosthetic device. Such indicators can include a marking that corresponds to another marking on the prosthetic device, a user application on a cellular phone or other mobile device, or an LED.

In the example embodiment of FIG. 7, coils 704 are arranged substantially in the same plane with each coil 704 partially overlapping an adjacent coil 704 to provide for better coverage in the transmission of the magnetic field. Each coil 704 can include a printed circuit board (PCB) trace or flexible wire on flexible circuit 708. Such a construction can generally allow EM transmitter 702 to be flexible enough to wrap around a portion of the prosthetic device.

EM transmitter 702 also includes circuitry 722 which is configured to receive power from a power supply via power cord 720. Circuitry 722 is electrically connected to each of coils 704 via traces 716 and 718 to deliver power to coils 704 for generating a magnetic field.

FIG. 8 provides a side view of EM transmitter 802 that is capable of being wrapped around a prosthetic device and includes overlapping flexible circuits 808 and 822 according to an embodiment. As shown in FIG. 8, EM transmitter 802 includes attachment portions 810 and 812 for forming a loop with EM transmitter 802 so that EM transmitter 802 can be worn around the prosthetic device. Attachment portions 810 and 812 can include Velcro, a magnet, a clip, a strap, a buckle, or other ways of securing EM transmitter 802 onto itself.

In one embodiment, one or both of attachment portions 810 and 812 can include a magnet that can be used to secure EM transmitter 802 onto a prosthetic device. The magnet may also be used to properly align EM transmitter 802 laterally or vertically onto the prosthetic device by securing the attachment portion onto a corresponding magnet located near an EM receiver of the prosthetic device. Such alignment of EM transmitter 802 can help to ensure a more efficient alignment of coils 804 with respect to the coils of an EM receiver of the prosthetic device. Other embodiments may use different alignment indicators such as a marking that corresponds to another marking on the prosthetic device or an LED to indicate when EM transmitter 702 is properly aligned with respect to an EM receiver of the prosthetic device.

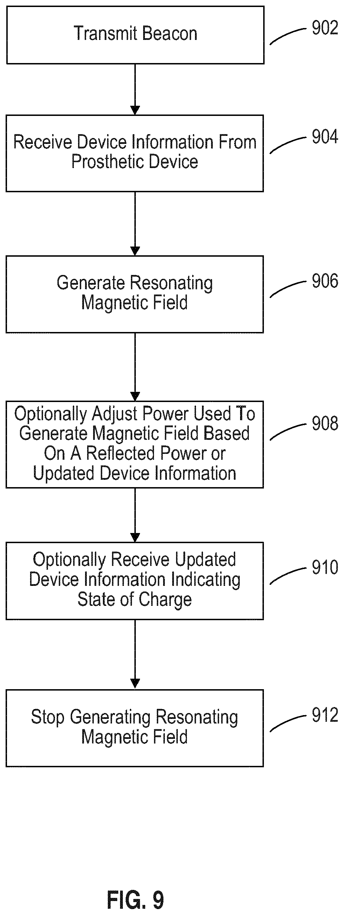

As shown in FIG. 8, EM transmitter 802 includes a top plurality of coils 804 and a bottom plurality of coils 824 each arranged on flexible circuits 808 and 822, respectively. Other embodiments may include more than the two layers of flexible circuits shown in FIG. 9.

Although there is a small lateral gap between each coil of coils 804 and each coil of coils 824, the coils are arranged such that the coils of flexible circuit 808 are laterally offset from the coils of flexible circuit 822 so as to provide increased coverage in transmitting a magnetic field. The coils of both flexible circuits may be connected to one another using the same traces or wiring on one of the flexible circuits or may use separate traces or wiring. EM transmitter 802 also includes circuitry 818 which receives power via power supplying circuit 820 and delivers power to coils 804 and 824.

FIG. 9 is a flowchart for a charging process which can be performed by EM transmitter 104 according to an embodiment. In block 902, circuitry of EM transmitter 104 transmits a beacon during a low power state to identify any devices such as prosthetic device 106 that can be wirelessly charged.

In block 904, circuitry of EM transmitter 104 receives device information in response to the beacon. As discussed above, the device information can include information about a prosthetic device such as identifying information, particular frequencies that the device can tune to, an average power usage of the device, or information about its power storage unit. After receiving the device information, EM transmitter 104 may exit its low power state and enter a transmission state for charging a prosthetic device such as prosthetic device 206 in FIG. 2.

In other embodiments, blocks 902 and 904 may be omitted such that EM transmitter 104 does not transmit a beacon or receive device information before generating a magnetic field. In such embodiments, EM transmitter 104 may instead periodically generate a magnetic field and measure a level of reflected power to determine whether there is a device within an effective range that can be charged. In other embodiments, EM transmitter 104 may continuously generate a magnetic field without entering a low power state.

In block 906, coils of EM transmitter 104 generate resonating magnetic field 124 at a frequency that can be based on the device information received in block 904. In some embodiments, the frequency is within the range of 100 kHz and 10 MHz. Circuitry of EM transmitter 104 may also set in block 906 an initial power used from a power supply for generating the magnetic field.

In block 908, circuitry of EM transmitter 104 adjusts the power used to generate the magnetic field based on a reflected power or updated device information. The reflected power may be expressed as a proportion of the power used to generate the magnetic field. As discussed above, the circuitry of EM transmitter 104 may increase the power used if the reflected power decreases since this may indicate that additional devices are charging with the magnetic field. The circuitry of EM transmitter 104 may also temporarily increase the power used to generate the magnetic field if the reflected power increases since this may indicate that the prosthetic device is farther away from EM transmitter 104. This temporary increase in power can serve as a test to determine whether the prosthetic device is still within an effective range for charging.

EM transmitter 104 may also use updated device information received from prosthetic device 106 via a digital wireless communications link. The updated device information can indicate a position or charging efficiency of prosthetic device 106. If the updated device information indicates that prosthetic device 106 is far away or is not charging efficiently, EM transmitter 104 may increase the power used to generate the magnetic field.

In other embodiments, block 908 may be omitted such that the power used to generate the magnetic field could be a fixed power level.

In some implementations, the circuitry of EM transmitter 104 can determine in block 908 to stop generating the magnetic field if a reflected power reaches or exceeds a threshold or if the updated device information indicates that prosthetic device 106 is too far away or no longer charging. For example, a threshold for the reflected power can be a value such as 80% of the power used to generate the magnetic field. A reflected power greater than or equal to the threshold may indicate that prosthetic device 106 is too far away for charging. In other embodiments, block 908 may be omitted such that EM transmitter 104 does not enter a low power state but rather continues to generate a magnetic field regardless of the reflected power or the receipt of any updated device information.

In block 910, the circuitry of EM transmitter 104 optionally receives updated device information from prosthetic device 106 indicating a state of charge for prosthetic device 106. In this regard, prosthetic device 106 may periodically transmit updated device information indicating a current state of charge. EM transmitter 104 may then stop generating the magnetic field in block 912 in response to receiving device information indicating that prosthetic device 106 is fully charged.

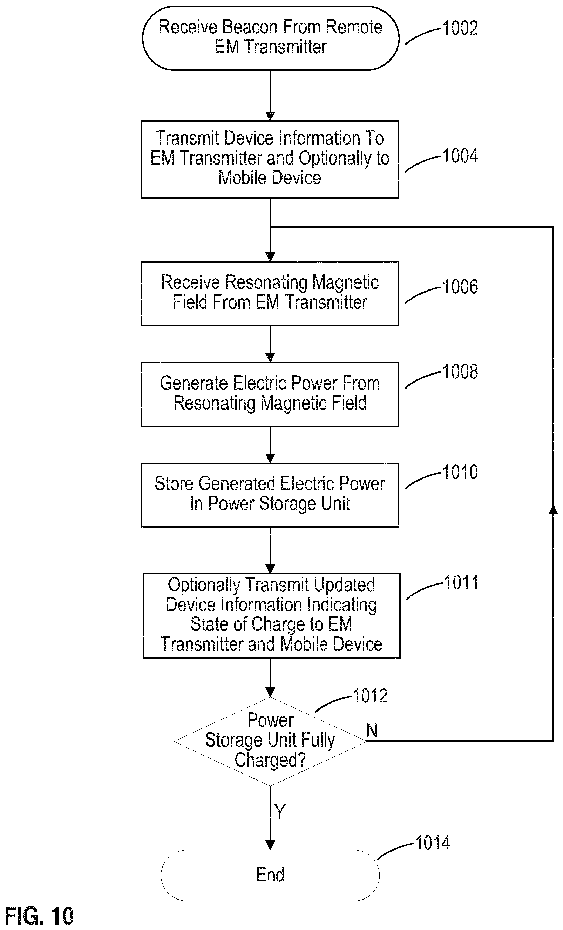

FIG. 10 is a flowchart for a charging process which can be performed by prosthetic device 106 of FIG. 1 according to an embodiment. The process begins in block 1002 when electronics 118 receives a beacon from a remote EM transmitter such as EM transmitter 104 to set up a wireless communications link between the EM transmitter and prosthetic device 106.

In block 1004, electronics 118 transmits device information to the EM transmitter via a wireless communications link using an antenna of electronics 118. Electronics 118 may also wirelessly transmit device information to a mobile device running an application for monitoring prosthetic device 106. The transmitted device information can include information about prosthetic device 106 such as identifying information, a resonant frequency or other frequencies that EM receiver 112 can tune to, an average power usage of prosthetic device 106, positioning or alignment information for charging, or information about BMS 114.

In other embodiments, blocks 1002 and 1004 may be omitted such that prosthetic device 106 does not receive a beacon from an EM transmitter or does not transmit device information.

In block 1006, coils of EM receiver 112 receive a resonating magnetic field from the remote EM transmitter. As discussed above, coils of EM receiver 112 are magnetically coupled with the EM transmitter at a frequency so as to allow for less alignment between the remote EM transmitter and EM receiver 112.

In block 1008, coils of EM receiver 112 generate electric power from the resonating magnetic field. In block 1010, the generated electric power is converted from AC power to DC power using BMS 114 and the converted DC power is stored in power storage unit 116 of BMS 114.

In block 1011, electronics 118 optionally transmits updated device information to the EM transmitter and a mobile device. The updated device information can indicate a current state of charge, a position or alignment, or a charging efficiency for prosthetic device 106.

In block 1012, electronics 118 determines whether power storage unit 116 is fully charged. If so, the charging process of FIG. 10 ends in block 1014. On the other hand, if it is determined in block 1012 that power storage unit 116 is not fully charged, the charging process of FIG. 10 returns to block 1006 to continue to receive the resonating magnetic field generated by the remote EM transmitter.

By magnetically coupling the EM transmitter with EM receiver 112 at a resonant frequency of EM receiver 112, it is ordinarily possible to wirelessly charge prosthetic device 106 without maintaining a tight alignment between the EM transmitter and EM receiver 112. This can generally allow for a user of prosthetic device 106 to freely move prosthetic device 106 while it is charging without having to remove prosthetic device 106. In addition, such wireless charging ordinarily allows for prosthetic device 106 to be better sealed from environmental conditions by not needing an exterior electrical connection for charging, which may otherwise require removal of an exterior cover while charging. Furthermore, EM resonant, wireless charging can allow for simultaneous charging of multiple prosthetic devices.

Those of ordinary skill in the art will appreciate that the various illustrative logical blocks, modules, and processes described in connection with the examples disclosed herein may be implemented as electronic hardware, computer software, or combinations of both. Furthermore, the foregoing processes can be embodied on a computer readable medium which causes a processor or computer to perform or execute certain functions.

To clearly illustrate this interchangeability of hardware and software, various illustrative components, blocks, and modules have been described above generally in terms of their functionality. Whether such functionality is implemented as hardware or software depends upon the particular application and design constraints imposed on the overall system. Those of ordinary skill in the art may implement the described functionality in varying ways for each particular application, but such implementation decisions should not be interpreted as causing a departure from the scope of the present disclosure.

The various illustrative logical blocks, units, modules, and controllers described in connection with the examples disclosed herein may be implemented or performed with a general purpose processor, a digital signal processor (DSP), an ASIC, a wireless communication chipset, a field programmable gate array (FPGA) or other programmable logic device, discrete gate or transistor logic, discrete hardware components, or any combination thereof designed to perform the functions described herein. A general purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The foregoing description of the disclosed example embodiments is provided to enable any person of ordinary skill in the art to make or use the embodiments in the present disclosure. Various modifications to these examples will be readily apparent to those of ordinary skill in the art, and the principles disclosed herein may be applied to other examples without departing from the scope of the present disclosure. The described embodiments are to be considered in all respects only as illustrative and not restrictive.

**Claims**

1. A prosthetic device (206), comprising:

   a power storage unit to power the prosthetic device;
   an electromagnetic receiver (112) including a plurality of coils (222) arranged about a portion of the prosthetic device, the electromagnetic receiver (112) configured to receive a magnetic field from an electromagnetic transmitter magnetically coupled with the electromagnetic receiver and to generate electric power from the magnetic field, wherein each coil of the plurality of coils is arranged adjacent to another coil of the plurality of coils such that diameters of the plurality of coils completely surround the portion of the prosthetic device; and
   circuitry configured to store the electric power generated from the magnetic field in the power storage unit.

2. The prosthetic device of Claim 1, wherein at least one coil of the plurality of coils partially overlaps an adjacent coil of the plurality of coils.

3. The prosthetic device of Claim 1, further comprising:

   a first flexible circuit including coils of the plurality of coils; and
   a second flexible circuit including coils of the plurality of coils, wherein the second flexible circuit is substantially parallel to the first flexible circuit.

4. The prosthetic device of Claim 3, wherein the coils of the first flexible circuit are laterally offset from the coils of the second flexible circuit.

5. The prosthetic device of Claim 1, wherein the magnetic field is a resonating magnetic field with a resonant frequency of the electromagnetic receiver.

6. The prosthetic device of Claim 1, wherein the electromagnetic receiver (112) is further configured to simultaneously receive multiple magnetic fields from different electromagnetic transmitters magnetically coupled with the electromagnetic receiver and to generate electric power from the simultaneously received magnetic fields.

7. The prosthetic device of Claim 1, further comprising electronics configured to:

   receive a beacon from the electromagnetic transmitter (1002) identifying the electromagnetic transmitter;
   transmit device information to the electromagnetic transmitter (1004), the device information related to at least one of identifying the prosthetic device, a frequency for magnetically coupling with the electromagnetic receiver, an average power usage of the prosthetic device, a charge efficiency, information about the power storage unit, or a proximity or alignment of the electromagnetic receiver with respect to the electromagnetic transmitter.

8. A system comprising the prosthetic device of Claim 1 and the electromagnetic transmitter magnetically coupled with the electromagnetic receiver.

9. The system of Claim 8, wherein the electromagnetic transmitter comprises:

   transmitter circuitry configured to receive electric power from a power supply; and
   a plurality of transmitter coils configured to generate a magnetic field using the electric power to magnetically couple with the electromagnetic receiver of the prosthetic device.

10. The system of Claim 9, wherein the plurality of transmitter coils is constructed to wrap around an exterior portion of the prosthetic device.

11. The system of Claim 9, wherein at least one coil of the plurality of transmitter coils partially overlaps an adjacent coil of the plurality of transmitter coils.

12. The system of Claim 8, wherein the electromagnetic transmitter comprises:

   a flexible circuit including a plurality of transmitter coils; and
   an attachment portion for forming a loop with the flexible circuit.

13. The system of Claim 8, wherein the electromagnetic transmitter comprises:

   a first flexible circuit including transmitter coils of a plurality of transmitter coils; and
   a second flexible circuit including transmitter coils of the plurality of transmitter coils,
   wherein the second flexible circuit is substantially parallel to the first flexible circuit.

14. The system of Claim 8, wherein the electromagnetic transmitter includes transmitter circuitry configured to initiate a low power state of the electromagnetic transmitter based on an amount of reflected power or updated device information received from the prosthetic device.

15. The system of Claim 8, wherein the electromagnetic transmitter includes transmitter circuitry configured to:

   receive device information from the prosthetic device (904); and
   set a frequency for generating the magnetic field (906) based on the device information.


**Patentansprüche**

1. Prothesevorrichtung (206), die Folgendes umfasst:

   eine Stromspeichereinheit zum Speisen der Prothesevorrichtung;
   einen elektromagnetischen Empfänger (112) mit mehreren Spulen (222), die um einen Teil der Prothesevorrichtung herum angeordnet sind, wobei der elektromagnetische Empfänger (112) zum Empfangen eines Magnetfeldes von einem elektromagnetischen Sender konfiguriert ist, der magnetisch mit dem elektromagnetischen Empfänger gekoppelt ist und zum Erzeugen von elektrischem Strom von dem Magnetfeld dient, wobei jede Spule der mehreren Spulen neben einer anderen Spule der mehreren Spulen angeordnet ist, so dass Durchmesser der mehreren Spulen den Teil der Prothesevorrichtung vollständig umgeben; und
   eine Schaltung, die zum Speichern des von dem Magnetfeld erzeugten elektrischen Stroms in der Stromspeichereinheit konfiguriert ist.

2. Prothesevorrichtung nach Anspruch 1, wobei wenigstens eine Spule der mehreren Spulen eine benachbarte Spule der mehreren Spulen teilweise überlappt.

3. Prothesevorrichtung nach Anspruch 1, die ferner Folgendes umfasst:

   eine erste flexible Schaltung mit Spulen der mehreren Spulen; und
   eine zweite flexible Schaltung mit Spulen der mehreren Spulen, wobei die zweite flexible Schaltung im Wesentlichen parallel zu der ersten flexiblen Schaltung ist.

4. Prothesevorrichtung nach Anspruch 3, wobei die Spulen der ersten flexiblen Schaltung seitlich von den Spulen der zweiten flexiblen Schaltung versetzt sind.

5. Prothesevorrichtung nach Anspruch 1, wobei das Magnetfeld ein Resonanzmagnetfeld mit einer Resonanzfrequenz des elektromagnetischen Empfängers ist.

6. Prothesevorrichtung nach Anspruch 1, wobei der elektromagnetische Empfänger (112) ferner zum gleichzeitigen Empfangen von mehreren Magnetfeldern von verschiedenen elektromagnetischen Sendern, die magnetisch mit dem elektromagnetischen Empfänger gekoppelt sind, und zum Erzeugen von elektrischem Strom von den gleichzeitig empfangenen Magnetfeldern konfiguriert ist.

7. Prothesevorrichtung nach Anspruch 1, die ferner Elektronik umfasst, konfiguriert zum:

   Empfangen einer Bake von dem elektromagnetischen Sender (1002), die den elektromagnetischen Sender identifiziert;
   Senden von Vorrichtungsinformationen zu dem elektromagnetischen Sender (1004), wobei sich die Vorrichtungsinformationen auf wenigstens eines aus einer Identifikation der Prothesevorrichtung, einer Frequenz zum magnetischen Koppeln mit dem elektromagnetischen Empfänger, einem durchschnittlichen Stromverbrauch der Prothesevorrichtung, einer Ladungseffizienz, Informationen über die Stromspeichereinheit oder einer Nähe oder Ausrichtung des elektromagnetischen Empfängers mit Bezug auf den elektromagnetischen Sender beziehen.

8. System, das die Prothesevorrichtung nach Anspruch 1 und den mit dem elektromagnetischen Empfänger magnetisch gekoppelten elektromagnetischen Sender umfasst.

9. System nach Anspruch 8, wobei der elektromagnetische Sender Folgendes umfasst:

   eine Senderschaltung, konfiguriert zum Empfangen von elektrischem Strom von einer Stromversorgung; und
   mehrere Senderspulen, konfiguriert zum Erzeugen eines Magnetfeldes mit Hilfe des elektrischen Stroms, zum magnetischen Koppeln mit dem elektromagnetischen Empfänger der Prothesevorrichtung.

10. System nach Anspruch 9, wobei die mehreren Senderspulen zum Wickeln um einen Außenabschnitt der Prothesevorrichtung konstruiert sind.

11. System nach Anspruch 9, wobei wenigstens eine Spule der mehreren Senderspulen eine benachbarte Spule der mehreren Senderspulen teilweise überlappt.

12. System nach Anspruch 8, wobei der elektromagnetische Sender Folgendes umfasst:

   eine flexible Schaltung mit mehreren Senderspulen; und
   einen Befestigungsabschnitt zum Bilden einer Schleife mit der flexiblen Schaltung.

13. System nach Anspruch 8, wobei der elektromagnetische Sender Folgendes umfasst:

   eine erste flexible Schaltung mit Senderspulen von mehreren Senderspulen; und
   eine zweite flexible Schaltung mit Senderspulen der mehreren Senderspulen, wobei die zweite flexible Schaltung im Wesentlichen parallel zu der ersten flexiblen Schaltung ist.

14. System nach Anspruch 8, wobei der elektromagnetische Sender eine Senderschaltung beinhaltet, die zum Einleiten eines Niedrigstromzustands des elektromagnetischen Senders auf der Basis einer Menge an reflektierter Leistung oder von aktualisierten Vorrichtungsinformationen wie von der Prothesevorrichtung empfangen konfiguriert ist.

15. System nach Anspruch 8, wobei der elektromagnetische Sender eine Senderschaltung aufweist, die konfiguriert ist zum:

   Empfangen von Vorrichtungsinformationen von der Prothesevorrichtung (904); und
   Einstellen einer Frequenz zum Erzeugen des Magnetfeldes (906) auf der Basis der Vorrichtungsinformationen.

**Revendications**

1. Dispositif prothétique (206), comprenant :

   une unité de stockage d'énergie pour alimenter le dispositif prothétique ;
   un récepteur électromagnétique (112) comportant une pluralité de bobines (222) disposées autour d'une partie du dispositif prothétique, le récepteur électromagnétique (112) étant configuré pour recevoir un champ magnétique provenant d'un émetteur électromagnétique couplé magnétiquement au récepteur électromagnétique et pour générer de l'énergie électrique à partir du champ magnétique, dans lequel chaque bobine de la pluralité de bobines est disposée adjacente à une autre bobine de la pluralité de bobines de telle façon que les diamètres de la pluralité de bobines entourent complètement la partie du dispositif prothétique ; et
   un circuit configuré pour stocker l'énergie électrique générée à partir du champ magnétique dans l'unité de stockage d'énergie.

2. Dispositif prothétique selon la revendication 1, dans lequel au moins une bobine de la pluralité de bobines chevauche partiellement une bobine adjacente de la pluralité de bobines.

3. Dispositif prothétique selon la revendication 1, comprenant en outre :

   un premier circuit souple comportant des bobines de la pluralité de bobines ; et
   un deuxième circuit souple comportant des bobines de la pluralité de bobines, le deuxième circuit souple étant pratiquement parallèle au premier circuit souple.

4. Dispositif prothétique selon la revendication 3, dans lequel les bobines du premier circuit souple sont décalées latéralement par rapport aux bobines du deuxième circuit souple.

5. Dispositif prothétique selon la revendication 1, dans lequel le champ magnétique est un champ magnétique résonant ayant une fréquence de résonance du récepteur électromagnétique.

6. Dispositif prothétique selon la revendication 1, dans lequel le récepteur électromagnétique (112) est en outre configuré pour recevoir simultanément de multiples champs magnétiques provenant de différents émetteurs électromagnétiques couplés magnétiquement au récepteur électromagnétique et pour générer de l'énergie électrique à partir des champs magnétiques reçus simultanément.

7. Dispositif prothétique selon la revendication 1, comprenant en outre une électronique configurée pour :

   recevoir de l'émetteur électromagnétique (1002) une balise identifiant l'émetteur électromagnétique ;
   transmettre une information de dispositif à l'émetteur électromagnétique (1004), l'information de dispositif concernant l'une au moins parmi une identification du dispositif prothétique, une fréquence de couplage magnétique avec le récepteur électromagnétique, une consommation d'énergie moyenne du dispositif prothétique, un rendement de charge, une information sur l'unité de stockage d'énergie, ou une proximité ou un alignement du récepteur électromagnétique par rapport à l'émetteur électromagnétique.

8. Système comprenant le dispositif prothétique selon la revendication 1 et l'émetteur électromagnétique couplé magnétiquement au récepteur électromagnétique.

9. Système selon la revendication 8, dans lequel l'émetteur électromagnétique comprend :

   un circuit émetteur configuré pour recevoir de l'énergie électrique à partir d'une alimentation en énergie ; et
   une pluralité de bobines émettrices configurées pour générer un champ magnétique en utilisant l'énergie électrique pour le couplage magnétique avec le récepteur électromagnétique du dispositif prothétique.

10. Système selon la revendication 9, dans lequel la pluralité de bobines émettrices est construite de façon à s'enrouler autour d'une partie extérieure du dispositif prothétique.

11. Système selon la revendication 9, dans lequel au moins une bobine de la pluralité de bobines émettrices chevauche partiellement une bobine adjacente de la pluralité de bobines émettrices.

**12.** Système selon la revendication 8, dans lequel l'émetteur électromagnétique comprend :

un circuit souple comportant une pluralité de bobines émettrices ; et
une partie de fixation pour former une boucle avec le circuit souple.

**13.** Système selon la revendication 8, dans lequel l'émetteur électromagnétique comprend :

un premier circuit souple comportant des bobines émettrices d'une pluralité de bobines émettrices ; et
un deuxième circuit souple comportant des bobines émettrices de la pluralité de bobines émettrices, le deuxième circuit souple étant pratiquement parallèle au premier circuit souple.

**14.** Système selon la revendication 8, dans lequel l'émetteur électromagnétique comporte un circuit émetteur configuré pour déclencher un état de faible puissance de l'émetteur électromagnétique sur la base d'une quantité de puissance réfléchie ou d'une information de dispositif actualisée reçue du dispositif prothétique.

**15.** Système selon la revendication 8, dans lequel l'émetteur électromagnétique comporte un circuit émetteur configuré pour :

recevoir une information de dispositif du dispositif prothétique (904) ; et
fixer une fréquence pour générer le champ magnétique (906) sur la base de l'information de dispositif.

Power Supply
102

EM Transmitter
104

124

106

EM Receiver 112

BMS 114

116

Electronics 118

FIG. 1

FIG. 2

FIG. 3

FIG. 4

FIG. 5

FIG. 6

**FIG. 7**

**FIG. 8**

EP 2 842 521 B1

| Transmit Beacon | — 902 |

| Receive Device Information From Prosthetic Device | — 904 |

| Generate Resonating Magnetic Field | — 906 |

| Optionally Adjust Power Used To Generate Magnetic Field Based On A Reflected Power or Updated Device Information | — 908 |

| Optionally Receive Updated Device Information Indicating State of Charge | — 910 |

| Stop Generating Resonating Magnetic Field | — 912 |

**FIG. 9**

Receive Beacon From Remote EM Transmitter —1002

Transmit Device Information To EM Transmitter and Optionally to Mobile Device —1004

Receive Resonating Magnetic Field From EM Transmitter —1006

Generate Electric Power From Resonating Magnetic Field —1008

Store Generated Electric Power In Power Storage Unit —1010

Optionally Transmit Updated Device Information Indicating State of Charge to EM Transmitter and Mobile Device —1011

—1012
Power Storage Unit Fully Charged?

N

Y

End —1014

FIG. 10

**EP 2 842 521 B1**

**Patent documents cited in the description**

- US 20110060421 A
- US 20120161544 A